# EUROPEAN PATENT APPLICATION

(11) **EP 4 442 359 A2**
(43) Date of publication of application: **09.10.2024**
(21) Application number: 22900501.2
(22) Date of filing: 30.11.2022
(51) Int. Cl.: B01J 19/28, C12M 1/00

(54) **IN-VITRO BIOSYNTHESIS REACTION APPARATUS AND IN-VITRO BIOSYNTHESIS METHOD**

(30) Priority: 30.11.2021 CN 202111448968; 26.01.2022 CN 202210093573; 26.01.2022 CN 202210095041; 26.01.2022 CN 202210093587; 11.03.2022 CN 202220528915 U; 11.03.2022 CN 202210239438
(71) Applicant: Kangma-Healthcode (Shanghai) Biotech Co., Ltd, Pudong New Area Shanghai 201321 (CN)
(72) Inventor: GUO, Min, Shanghai 201321 (CN); CHEN, Wenfang, Shanghai 201321 (CN); YU, Xue, Shanghai 201321 (CN)
(74) Representative: Murgitroyd & Company
(86) International application number: PCT/CN2022/135274
(87) International publication number: WO 2023/098697

(57) **Abstract**

An in-vitro biosynthesis reaction apparatus and an in-vitro biosynthesis method. The in-vitro biosynthesis reaction apparatus comprises: a reaction body (10), which is used for containing a protein synthesis reaction liquid, so as to carry out an in-vitro biosynthesis reaction, wherein an opening part (10a) is arranged on the reaction body (10), and the opening part (10a) is provided with a feeding port used for enabling the synthesis reaction liquid to enter the reaction body (10) and a discharging port allowing a reaction product to be discharged; and a partition part (20), which is used for incomplete separation of the internal space of the reaction body (10).

## Description

### TECHNICAL FIELD

The present disclosure relates to the field of biotechnology, and in particular to a reaction device for in-vitro biosynthesis and an in-vitro biosynthesis method.

### BACKGROUND

Various in-vitro biosyntheses, compared with in-vivo synthesis, such as in-vitro cell-free protein synthesis (in-vitro protein synthesis, in-vitro cell-free protein synthesis), which mainly depends on cell-free expression system, and uses exogenous target DNA as a template for protein synthesis, and artificially control the addition of substrates required for protein synthesis and cofactors of transcription and translation-related proteins. That is, the in-vitro synthesis of the target protein is achieved through the reaction liquid.

However, at present, the in-vitro synthesis is performed on a small scale in laboratories, and there is no production device that can achieve expansion.

It is urgent to develop a production expansion device that can not only greatly expand the volume, but also maintain the reactivity to the maximum extent.

### SUMMARY

The present disclosure provides following technical solutions to solve a problem that current in-vitro biosynthesis cannot be scaled up for production.

The present disclosure provides a reaction device for in-vitro biosynthesis, including a reaction chamber body configured to accommodate reaction liquid for synthesis to perform an in-vitro biosynthesis reaction, the reaction chamber body being provided with an opening portion having a feeding port configured to allow the reaction liquid for synthesis to enter the reaction chamber body and a discharging port configured to allow a reacted product to be discharged therefrom; and a partition portion configured to incompletely partition an internal space of the reaction chamber body.

The reaction device according to the present disclosure further has the following feature: the reaction chamber body is a tank body, preferably a horizontal tank body, and the tank body is spherical or ellipsoidal.

The reaction device according to the present disclosure further has the following feature: the partition portion includes at least one partition member, preferably, the partition member is in a form of a sheet.

The reaction device according to the present disclosure further has the following feature: a plurality of partition members are evenly spaced apart.

The reaction device according to the present disclosure further has the following feature: a length of the partition member extends in a form of a curved line or a folded line.

The reaction device according to the present disclosure further has the following feature: an angle a is formed between a connecting line formed between two ends of the length of the partition member and a central axis of an internal space of the reaction chamber body in the same direction as an extending direction of the length, preferably, the angle a is less than or equal to 60°, preferably, less than or equal to 45°, and further preferably, less than or equal to 20°.

The reaction device according to the present disclosure further has the following feature: at least one partition member is rotatably and/or non-rotatably fixed on an inner wall of the reaction chamber body.

The reaction device according to the present disclosure further has the following feature: when the partition portion is provided on the inner wall, one side edge of the sheet-shaped partition member faces an inner wall of the reaction chamber body.

The reaction device according to the present disclosure further has the following feature: when the partition portion is provided on the inner wall, one side edge of the sheet-shaped partition member faces an inner wall of the reaction chamber body, and another side edge of the partition member away from the inner wall is provided with a recessed area.

The reaction device according to the present disclosure further has the following feature: when one side edge of the sheet-shaped partition member faces the inner wall of the reaction chamber body, an angle between a sheet-shaped surface of the partition member and the opposite inner wall is not equal to 90°.

The reaction device according to the present disclosure further has the following feature: in use, the reaction chamber body is rotated to perform the reaction, preferably, a horizontal central axis of the reaction chamber body is deviated from a horizontal state.

The reaction device according to the present disclosure further has the following feature: the partition member is provided with at least one flow hole. Preferably, the at least one flow hole on the partition member is evenly distributed.

The reaction device according to the present disclosure further has the following feature: when the partition member is provided on the inner wall, at least one discontinuity is provided between the partition member and the inner wall. Preferably, the at least one discontinuity corresponding to the partition member is equally spaced apart.

The reaction device according to the present disclosure further has the following feature: the opening portion is further provided with a ventilation unit having a gas inlet and/or a gas outlet, the gas inlet is configured to allow an external gas to enter an internal space of the reaction chamber body, and the gas outlet is configured to allow the gas in the internal space to be exhausted; preferably, one or more of the feeding port, the discharging port, the gas inlet, and the gas outlet are the same opening.

The reaction device according to the present disclosure further has the following feature: the reaction device further includes a gas intake unit and/or a gas exhaust unit, wherein the gas inlet allows the external gas to enter the internal space through the gas intake unit, and the gas outlet allows gas in the internal space to be exhausted from the gas exhaust unit through the exhaust unit.

The reaction device according to the present disclosure further has the following feature: the gas intake unit includes at least one gas intake duct having an end facing the gas inlet to deliver the external air to the internal space, or an end of the gas intake duct extends into the internal space from the gas inlet, at this time, preferably, at least one gas intake duct extends along a horizontal direction of the reaction chamber body; preferably, a duct wall of a portion of the at least one gas intake duct that enters the internal space is provided with at least one gas exhaust hole, preferably, the at least one gas exhaust hole is evenly distributed, and/or one end of the gas intake duct entering the internal space is sealed.

The reaction device according to the present disclosure further has the following feature: the gas exhaust unit includes a gas exhaust duct, preferably, a portion of the gas intake duct between two ends of the gas intake duct is provided in the gas exhaust duct, one end of the gas intake duct extends out of one end of the gas exhaust duct and extends into the internal space, and another end of the gas intake duct extends out of another end of the gas exhaust duct and is in communication with an air source.

The reaction device according to the present disclosure further has the following feature: the reaction device further includes a cover assembly having a cover body adapted to the feeding port and configured to cover the feeding port. Preferably, the cover assembly further includes a sealing ring, a tank cover latch, and a locking handle.

The reaction device according to the present disclosure further has the following feature: when the reaction device includes a cover assembly, the gas exhaust unit further includes a gas exhaust hole provided on the cover body, and the gas exhaust duct is in communication with the internal space through the gas exhaust hole.

The reaction device according to the present disclosure further has the following feature: the reaction device further includes a rotating portion configured to rotate the reaction chamber body, preferably, the rotating portion is rotatably connected to the reaction chamber body to rotate the reaction chamber body; and/or the rotating portion is further provided with a rotating wheel, the reaction chamber body is provided on the rotating wheel, and the rotating wheel rotate along with a rotation of the reaction chamber body.

The reaction device according to the present disclosure further has the following feature: the rotating portion includes a driving unit that drives the reaction chamber body to rotate.

The reaction device according to the present disclosure further has the following feature: the reaction device further includes a temperature regulating device configured to maintain the reaction under a predetermined temperature condition; preferably, the temperature regulating device is configured to regulate a temperature of gas entering the reaction chamber body to maintain the predetermined temperature condition.

The reaction device according to the present disclosure further has the following feature: the reaction device further includes a housing adapted to the reaction chamber body and configured to accommodate the reaction chamber body.

Preferably, the housing has any one or a combination of the following features:
(1) The housing is provided with an opening and closing door, and preferably, the opening and closing door is provided with a vent hole;
(2) A display screen is provided on an outer side of the housing, and preferably, the display screen is located on an outer surface of an upper top of the housing;
(3) An outer side of the housing is provided with an object placing platform configured to place an object, preferably, an object placing surface of the object placing platform is provided with non-slip textures, and more preferably, an outer surface of an upper top of the housing is the object placing platform;
(4) The housing is provided with any one or more of a gas intake channel configured to allow external gas to enter the internal space of the reaction chamber body, a gas exhaust channel configured to allow gas in the internal space of the reaction chamber body to be exhausted, a feeding channel configured to allow the reaction liquid for synthesis to enter the internal space of the reaction chamber body to perform the in-vitro biosynthesis reaction, and a discharging channel through which the reacted product is discharged.

The present disclosure also provides an in-vitro biosynthesis method, including useing the aforementioned reaction device to accommodate the reaction liquid for synthesis to perform the in-vitro biosynthesis reaction.

The in-vitro biosynthesis method according to the present disclosure further has the following feature: a ratio of a volume of the reaction liquid for synthesis configured to perform the reaction to a volume of the reaction chamber body is less than or equal to 30%.

The in-vitro biosynthesis method according to the present disclosure further has the following feature: during the reaction, an external gas is introduced into the internal space and/or gas in the internal space is exhausted.

The in-vitro biosynthesis method according to the present disclosure further has the following feature: during the reaction, the reaction chamber body of the reaction device is rotated, and preferably, a speed of rotation is less than or equal to 120 r/min.

The present disclosure provides a bioreactor for an in-vitro biosynthesis reaction, which includes a reaction chamber body configured to accommodate reaction liquid for synthesis to perform an in-vitro protein synthesis reaction, the reaction chamber body is provided with an opening portion having a feeding port, a discharging port, and a ventilation unit, the feeding port is configured to allow the reaction liquid for synthesis to enter the reaction chamber body, the discharging port is configured to allow a reacted product to be discharged from the reaction chamber body, the ventilation unit is provided with a gas inlet and/or a gas outlet, the gas inlet is configured to allow external gas to enter an internal space of the reaction chamber body, and the gas outlet is configured to allow gas in the internal space to be exhausted, preferably, the reaction chamber body is a tank body.

The bioreactor according to the present disclosure further has the following feature: an inner wall of the reaction chamber body is provided with at least one first protrusion.

The bioreactor according to the present disclosure further has the following feature: one or more of the feeding port, the discharging port, the gas inlet, and the gas outlet are the same opening.

The bioreactor according to the present disclosure further has the following feature: the bioreactor further includes a rotation mounting portion configured to be rotatably connected to a rotating portion that rotates the reaction chamber body, preferably, the rotation mounting portion is located on an opposite side of the opening portion, and after the rotation mounting portion is rotatably connected to the rotating portion, the opening portion is inclined upwards.

The present disclosure also provides a partition member for a biological reaction, which is configured to incompletely partition an internal space of a reaction chamber body of the aforementioned bioreactor.

The bioreactor according to the present disclosure further has the following feature: a length of the partition member extends in a curved line or a folded line.

The bioreactor according to the present disclosure further has the following feature: the partition member is in a form of a sheet having two opposite sheet-shaped surfaces and side edges provided on both sides of the two opposite sheet-shaped surfaces.

The bioreactor according to the present disclosure further has the following feature: one of the side edges of the partition member is provided with a recessed area.

The bioreactor according to the present disclosure further has the following feature: the partition member is provided with at least one flow hole.

The bioreactor according to the present disclosure further has the following feature: an outer surface of the partition member is provided with at least one second protrusion.

The bioreactor according to the present disclosure further has the following feature: the partition member is integrally formed with the bioreactor.

The present disclosure also provides a housing adapted to the reaction chamber body and configured to accommodate the reaction chamber body.

The housing according to the present disclosure having any one or a combination of the following features:
(1) The housing is provided with an opening and closing door, and preferably, the opening and closing door is provided with a vent hole;
(2) A display screen is provided on an outer side of the housing, and preferably, the display screen is located on an outer surface of an upper top of the housing;
(3) An outer side of the housing is provided with an object placing platform configured to place an object, preferably, an object placing surface of the object placing platform is provided with non-slip textures, and more preferably, an outer surface of an upper top of the housing is the object placing platform;
(4) The housing is provided with any one or more of a gas intake channel configured to allow external gas to enter the internal space of the reaction chamber body, a gas exhaust channel configured to allow gas in the internal space of the reaction chamber body to be exhausted, a feeding channel configured to allow the reaction liquid for synthesis to enter the internal space of the reaction chamber body to perform the in-vitro biosynthesis reaction, and a discharging channel through which a reacted product is discharged.

The present disclosure also provides a reaction device, including the aforementioned bioreactor and at least one aforementioned partition member, the at least one partition member incompletely partitions an internal space of the reaction chamber body of the bioreactor, and the bioreactor is integrally formed with the partition member.

The reaction device according to the present disclosure further has the following feature: the reaction device further includes the aforementioned housing.

In addition, the in-vitro biosynthesis according to the present disclosure mainly refers to in-vitro biosynthesis such as in-vitro protein synthesis reaction and in-vitro mRNA synthesis reaction.

### Functions and effects of the present disclosure

The reaction device for in-vitro biosynthesis and the in-vitro biosynthesis method according to the present disclosure have at least the following functions and effects:
(1) The internal space of the reaction chamber body is incompletely partitioned by the partition portion, when the reaction liquid for in-vitro biosynthesis reaction is accommodated in the reaction chamber body, the reaction liquid in a "flowing state" can flow from one space area to another space area, and the reaction liquid flowing through the partition portion will be blocked by the partition portion to increase the mixing intensity of the reaction liquid during the in-vitro synthesis reaction, thereby improving the reaction efficiency.
(2) During the reaction process, the reaction chamber body is rotated, so that the reaction liquid can be effectively spread on the inner wall, and the in-vitro biosynthesis efficiency is increased;
(3) When the reaction chamber body is a tank body, compared with other shapes such as cuboid, since the inner wall has a radian, and there is no dead corner, it is not easy to generate foam during the reaction, which ensures the synthesis quality, and compared with other shapes, the same volume has a larger inner wall surface area, the reaction liquid can be spread thinner, which is more conducive to synthesis;
(4) The sheet-shaped partition portion increases a contact area of the reaction liquid, thereby increasing a degree of spreading of the reaction liquid;
(5) The partition portion extends in a curved line or a folded line, and in the same internal space, a contact area of the reaction liquid is also increased compared with a straight line, which is beneficial to the spreading of the reaction liquid;
(6) The gas inlet is configured to allow the external gas to enter the internal space of the reaction chamber body, so that oxygen-containing gas, such as air, can be introduced to facilitate the in-vitro biosynthesis reaction;
(7) The gas outlet is configured to allow gas in the internal space to be exhausted, so that the gas generated in the reaction process, such as ethanol, which is unfavorable to the reaction, can be exhausted in time, so as not to affect the synthesis efficiency and quality.

### BRIEF DESCRIPTION OF THE DRAWINGS

FIG. 1 is an overall structure schematic view of a first reaction device according to the present disclosure.
FIG. 2 is another schematic view of FIG. 1.
FIG. 3 is a structural schematic view of a reaction chamber body according to the present disclosure.
FIG. 4 is a structural schematic view of a reaction device of a first structure according to the present disclosure, showing only a partition portion.
FIG. 5 is a schematic view for explaining that an internal space of the reaction chamber body according to the present disclosure is partitioned.
FIG. 6 is a structural schematic view of the reaction chamber body in which the internal space of the reaction device of the first structure according to the present disclosure is incompletely partitioned by the partition portion.
FIG. 7 is a structural schematic view of a partition member of the present disclosure.
FIG. 8 is an overall structural schematic view of a reaction device of a second structure according to the present disclosure.
FIG. 9 is another schematic view of FIG. 8.
FIG. 10 is an assembly view of a gas intake unit, a gas exhaust unit, and a cover assembly according to the present disclosure.
FIG. 11 is an overall structure schematic view of a reaction device of a third structure according to the present disclosure.
FIG. 12 is a side view of FIG. 11.
FIG. 13 is a graph showing an experimental result of experimental example 1 according to the present disclosure.
FIG. 14 is a graph showing an experimental result of experimental example 2 of the present disclosure.

### DETAILED DESCRIPTION OF THE EMBODIMENTS

Embodiments of the present disclosure will now be described with reference to the accompanying drawings. For the specific methods or materials used in the embodiments, those skilled in the art may make conventional alternative selections on the basis of the technical idea of the present disclosure and according to the existing technology, and are not limited to the specific description of the embodiments of the present disclosure.

Herein, a direction in which a length of an axis of a reaction chamber body is longer is referred to as a "length direction" of the reaction chamber body, otherwise, it is referred to as a "width direction", for example, when a long axis of an ellipsoid is in a horizontal state, a horizontal direction is a length direction.

As used herein, the "length direction" refers to a substantially equivalent direction, rather than an absolutely consistent direction. Similarly, the "width direction" refers to a substantially equivalent direction.

### Embodiment 1

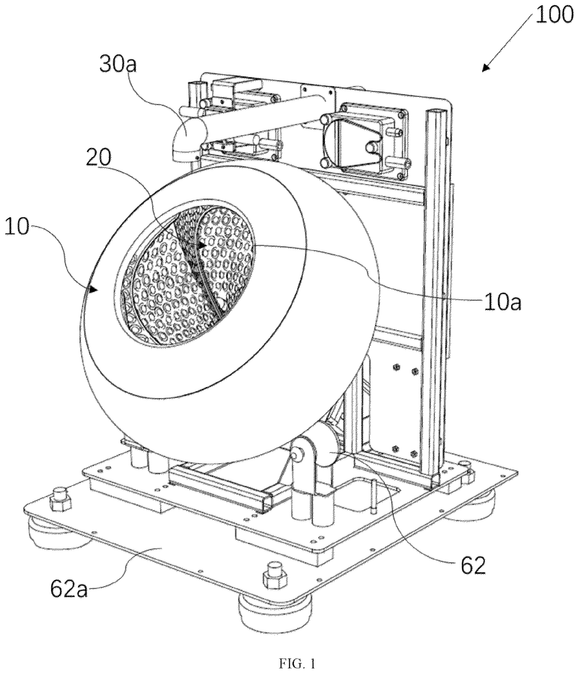

FIG. 1 is an overall structure schematic view of a first reaction device according to the present disclosure.

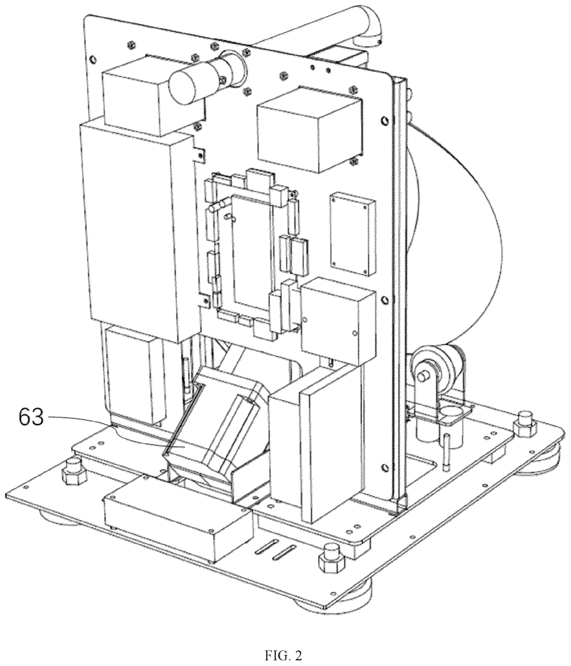

FIG. 2 is another schematic view of FIG. 1.

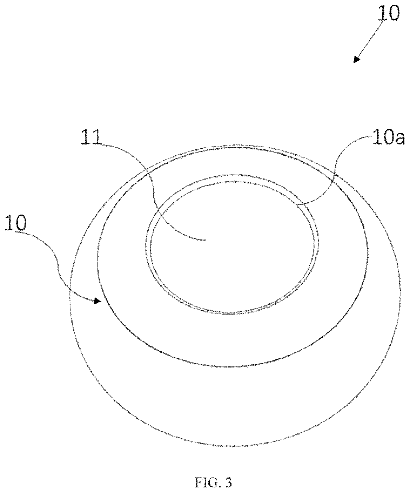

FIG. 3 is a structural schematic view of a reaction chamber body according to the present disclosure.

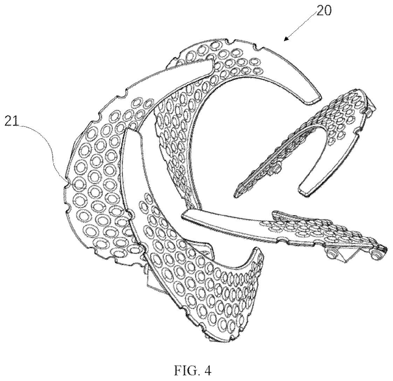

FIG. 4 is a structural schematic view of a reaction device of a first structure according to the present disclosure, showing only a partition portion.

As shown in FIG. 1 to FIG. 4, a reaction device 100 for in-vitro biosynthesis provided according to this embodiment includes a reaction chamber body 10 and a partition portion 20.

The reaction chamber body 10 is configured to accommodate reaction liquid for synthesis to perform an in-vitro biosynthesis reaction. The reaction chamber body 10 is provided with an opening portion 10a. The opening portion 10a has a feeding port configured to allow the reaction liquid for synthesis to enter an internal space 11 of the reaction chamber body to perform a reaction and a discharging port configured to discharge a reacted product. The reaction liquid may be poured directly into the feeding port, or the reacted product may be poured out from the discharging port. The feeding and discharging can also be achieved through other methods, for example, a feeding duct may be used to transport the reaction liquid for synthesis from the feeding port to the internal space 11, or a discharging duct may be used to discharge the reacted product from the internal space 11.

In an embodiment, the opening portion 10a also has a ventilation unit having a gas inlet and/or a gas outlet. The gas inlet is configured to allow external gas to enter the internal space of the reaction chamber body, so that oxygen-containing gas, such as air, can be introduced, which is advantageous for the in-vitro biosynthesis reaction. The gas outlet is configured to discharge the gas in the internal space, so that the gas generated during the reaction that is unfavorable to the reaction, such as ethanol, can be exhausted in time to avoid affecting the synthesis efficiency and quality.

In an embodiment, one or more of the feeding port, the discharging port, the gas inlet, and the gas outlet are the same opening. In FIG. 1, the feeding port, the discharging port, the gas inlet, and the gas outlet are the same opening, i.e., the opening portion 10a. In other embodiments, the feeding port, the discharging port, the gas inlet, and the gas outlet may be different openings, or some of them may be the same opening.

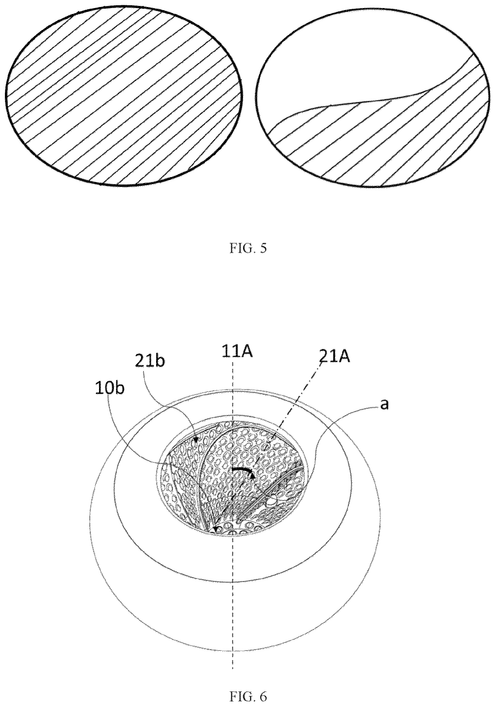

FIG. 5 is a schematic view for explaining that the internal space of the reaction chamber according to the present disclosure is divided.

In FIG. 5, a part covered by oblique lines represents a partition portion.

The partition portion 20 is configured to incompletely partition the internal space of the reaction chamber body 10.

Explanation is made with reference to FIG. 5, in which a circle represents a section of the reaction chamber body, an interior of the circle represents an internal space, and an arc in a middle of the circle represents the partition. The "incompletely partition" is relative to "completely partition". The "completely partition" means that the entire internal space is partitioned into different uncommunicated spaces. That is, the partition spaces are completely partitioned from each other, for example, two spaces are formed by completely partitioning by a plate along a certain cross-section of the entire internal space. At this time, for example, on a left side of FIG. 5, a cross-section of the entire partition completely covers a cross-section of the internal space. The "incompletely partition" means that the internal space is incompletely partitioned. As shown on the right side of in FIG. 5, although left and right edges and a lower edge of the partition are respectively in contact with an inner side wall, an upper edge is not in contact with the inner side wall, such incomplete partition forms space areas that are in communication with each other. Specifically, as shown on the right side of FIG. 5, on the cross-section, the part covered by the partition is the space area, and the space areas are in communication with each other through a blank part thereof.

In this way, due to the incompletely partition, when the aforementioned reaction liquid is contained, the reaction liquid in a "flowing state" can flow from one space area to another space area, and the reaction liquid flowing through the partition portion 20 will be blocked by the partition portion 20 to increase the mixing intensity of the reaction liquid during the in-vitro synthesis reaction, thereby improving the reaction efficiency.

The "flowing state" herein means that the reaction liquid is not stationary, but at least partially moves relative to an inner wall, and the reasons for this include, but are not limited to, the following:

First, it is caused only by the movement of the reaction chamber body 10, for example, only by the vibration, rotation, etc. of the reaction chamber body;

Second, it flows only by other external forces:
(a) For example, the reaction liquid is stirred by an external force, for example, the partition portion 20 is rotated to stir the reaction liquid, so that the reaction liquid enters the flowing state;
(b) For another example, the reaction liquid may be subjected to fluctuations caused by the introduction of high-pressure airflow or ultrasonic vibration, so that the reaction liquid enters the flowing state.

Third, both the first and the second work, for example, when the reaction chamber body 10 rotates, the partition portion 20 also rotates.

In addition, when the reaction liquid is in the flowing state, due to the blocking of the partition portion 20, another effect is that the chance of the reaction liquid being contact with the inner wall of the reaction chamber body 10 is increased, the reaction liquid being contact with the inner wall has a chance of being spread on the inner wall under certain conditions, such as gravity or rotation, and the spread reaction liquid has a larger surface area and can be more fully contact with the air, thereby improving the in-vitro synthesis efficiency.

In an embodiment, the reaction chamber body 10 is a tank body as shown in the figure. Since the tank body is spherical or ellipsoidal, compared with other shapes, a surface area of the inner wall is larger, the reaction liquid has more chances to be spread on the inner wall, and since there is no dead corner, it is not easy to generate foam during the reaction. Preferably, it is a horizontal tank body, that is, when the tank body is ellipsoidal, a long axis is in a horizontal direction, which avoids the deposition of the reaction liquid at the bottom and is more conducive to spreading than in the vertical direction.

In an embodiment, during use, the reaction chamber body 10 is rotated to complete the reaction, so that the reaction liquid can be effectively spread on the inner wall to increase the efficiency of the in-vitro synthesis, and the degree to which the reaction liquid is spread can be adjusted by controlling a volume of the added reaction liquid. Preferably, a horizontal central axis of the reaction chamber body 10 is deviated from a horizontal state by a certain angle, that is, the reaction chamber body 10 is inclined, so that when the reaction chamber body 10 rotates, the reaction liquid can be more easily spread on more portions of the inner wall.

FIG. 6 is a structural schematic view of the reaction chamber body in which the internal space of the reaction device of the first structure according to the present disclosure is incompletely partitioned by the partition portion.

In an embodiment, the inner wall of the reaction chamber body 10 is provided with at least one first protrusion 10b (as shown in FIG. 6). Through the first protrusion 10b, the surface area of the inner wall can be increased, thereby increasing a contact area between the inner wall and the reaction liquid, and increasing the degree to which the reaction liquid is spread.

In an embodiment, as shown in FIG. 4, the partition portion 20 includes at least one partition member 21, so as to incompletely partition the internal space 11 to form a plurality of space areas, and the plurality of partition members 21 can increase the chance of the reaction liquid being mixed and spread on the inner wall.

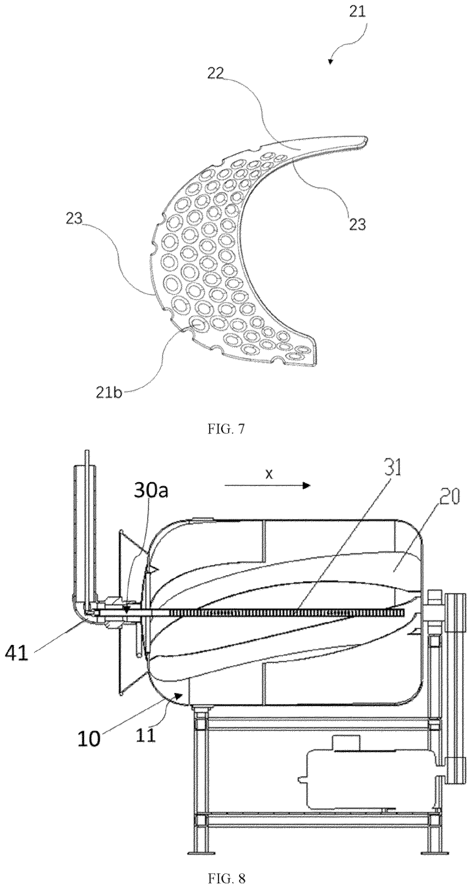

FIG. 7 is a structural schematic view of the partition member of the present disclosure.

As shown in FIG. 7, in an embodiment, the partition member 21 is in a form of a sheet, so that when the reaction liquid can be in contact with a sheet-shaped surface 22 of the partition member 21, the reaction liquid can also be spread onto the sheet-shaped surface 22. In this way, an area in which the reaction liquid of the same volume is spread is increased. Relatively speaking, the reaction liquid is also easier to be spread, and the contact area between the reaction liquid and the air is also increased, and the reaction efficiency is improved. The sheet-shaped surface 22 herein, as shown in FIG. 7, is relative to the side edge 23. In this case, the greater the number of partition members 21, the greater the area of the partition members 21 that can be in contact with the reaction liquid, and a spread area of the reaction liquid can be more increased, so that the contact area between the entire reaction liquid and the air is increased. Certainly, if the number of the partition members 21 is too large, a time for the spread reaction liquid to be contacted with the air each time may be too short, resulting in a longer reaction time, and if the number of the partition members 21 is too large, the overall weight is too large, which is not conducive to use, while the overall increase in the spreading is not too large, resulting in a waste of cost.

In an embodiment, the plurality of partition members 21 are evenly spaced apart, so that the internal space 11 can be incompletely partitioned into same space areas, and the spreading degree of the reaction liquid in each space area is equivalent. That is, the contact area of the reaction liquid is equivalent, the reaction efficiency is uniform, and the overall reaction effect is better.

In an embodiment, a length of the partition member 21 extends in a form of a curved line or a folded line, which can increase the chance of contact between the reaction liquid and the partition member 21 in the same internal space, especially when the partition member is in a form of a sheet, the contact area can be further increased, thereby improving the synthesis efficiency.

In an embodiment, as shown in FIG.6, an angle *a* is formed between a connecting line 21A formed between two ends of the length of the partition member 21 and a central axis 11A of the internal space 11 of the reaction chamber body 10 in the same direction as an extending direction of the length.

Herein, "the central axis 11A of the internal space 11 of the reaction chamber body 10 in the same direction as an extending direction of the length" specifically means that when the length of the partition member 21 extends substantially in a length direction of the reaction chamber body 10, the central axis 11A is a central axis in the length direction of the reaction chamber body 10. When the length of the partition member 21 extends substantially in a width direction of the reaction chamber body 10, the central axis 11A is a central axis in the width direction of the reaction chamber body 10. The same direction herein means substantially the same, not absolutely the same.

The angle *a* herein means that the line 21A where the connecting line is located is not parallel to the central axis 11A, but is at an angle to the central axis 11A, that is, the line 21A is inclined to the central axis 11A. Preferably, the angle *a* is less than or equal to 60°, preferably less than or equal to 45°, and further preferably less than or equal to 20°. In this way, it is convenient for the reaction liquid in each space area formed by the partition to flow from one end to the other end of the reaction chamber body 10. In this way, when an end of the partition member 21 is in communication with the adjacent space area, it is convenient for the reaction liquid to flow from the end to the adjacent space area, thereby improving the mixing efficiency. The angle *a* should not be too large, that is, a connecting line 21a should not deviate too much from the axis, otherwise the reaction liquid will accumulate at one end of the reaction chamber body 10, which is not conducive to spreading.

In an embodiment, the partition portion 20 is provided on the inner wall of the reaction chamber body 10. In practice, the partition portion 20 may be rotatably fixed on the inner wall or non-rotatably fixed on the inner wall. When a plurality of partition members 21 are provided, the partition portions 20 may be all rotatably provided on the inner wall or partially rotatably provided on the inner wall. Preferably, when the partition member 21 is in a form of a sheet, one side edge 23 of the sheet-shaped partition member 21 faces the inner wall of the reaction chamber body 10, that is, the partition member 21 is rotatably or non-rotatably provided on the inner wall by the rotatability or non-rotatability between the side edge 23 and the inner wall.

In an embodiment, when one side edge 23 of the sheet-shaped partition member 21 faces the inner wall of the reaction chamber body 10, an angle between a sheet-shaped surface of the sheet-shaped partition member and an opposite inner wall (referring to an inner wall where the side edge 23 is located) is not equal to 90°, which actually means that the sheet-shaped surface is not completely perpendicular to the inner wall, but deviates from a vertical plane by a certain angle *b.* In this way, compared with perpendicular, foam generated in the reaction can be reduced, the reaction effect can be improved, and the reaction liquid can be better spread.

In an embodiment, the length of the partition member 21 extends along the length direction of the reaction chamber body 10, and extends from one end to the other end in the length direction of the reaction chamber body, that is, the length of the partition member is substantially equivalent to the length of the reaction chamber body, which can ensure a larger contact area, thereby improving the synthesis efficiency.

In an embodiment, the partition member 21 is provided with at least one flow hole (not shown), so as to facilitate the reaction liquid to flow from one space area to another space area. Preferably, when a plurality of flow holes are provided, the flow holes on at least one partition member 21 are evenly arranged on the partition member 21, for example, three partition members 21 are provided, each partition member 21 is provided with a plurality of flow holes, and the flow holes on any one or more of the three partition members may be evenly distributed.

In an embodiment, when the partition member 21 is provided on the inner wall, at least one discontinuity is provided between the partition member and the inner wall, that is, at least one gap is provided between the partition member 21 and the inner wall, which facilitates the reaction liquid to flow from one space area to another space area. Preferably, the discontinuities between the at least one partition member 21 and the inner wall are equally spaced apart. For example, three partition members 21 are provided on the inner wall, each partition member 21 has at least one discontinuity with the inner wall, and the discontinuities between at least one of the three partition members 21 and the inner wall are equally spaced apart.

In an embodiment, as shown in FIGS. 6 and 7, an outer surface of the partition member 21 is provided with at least one second protrusion 21b. Similarly, the surface area of the partition member 21 can be increased by the second protrusion 21b, thereby increasing a contact area between the partition member 21 and the reaction liquid, and increasing the degree to which the reaction liquid is spread.

In an embodiment, the reaction device 100 further includes a gas intake unit. The aforementioned gas inlet allows external gas to enter the internal space 11 through the gas intake unit.

In an embodiment, the gas intake unit 30 includes at least one gas intake duct. As shown in FIG. 1, which shows one gas intake duct 30a, one end of which faces the gas inlet to deliver the external gas to the internal space, i.e., the gas intake duct has a distance from the internal space 11.

In an embodiment, the reaction device 100 further includes a gas exhaust unit, through which the aforementioned gas outlet allows the gas in the internal space 11 to be exhausted.

In an embodiment, the exhaust unit includes at least one gas exhaust duct that is in communication with the aforementioned gas outlet or extends into the internal space 11 to exhaust the gas from the internal space 11.

In an embodiment, the reaction device 100 further includes a rotating portion configured to rotate the reaction body 10. Preferably, the rotating portion is rotatably connected to the reaction chamber body to rotate the reaction chamber body 10. Preferably, the rotating portion is rotatably connected to a rotation mounting portion of the reaction chamber body 10 located on an opposite side of the opening portion. After the rotating connection, an opening of the opening portion is inclined upward (as shown in FIG. 1). In this way, the reaction liquid can be in better contact with and spread on the inner wall of the reaction chamber body 10 and the partition portion 20 during rotation, and the reaction liquid cannot overflow even if the opening is large. In this way, when the opening is used as a feeding port or a discharging port, it is more convenient for feeding or discharging, and when the opening is used as a gas inlet or a gas outlet, it can ensure a larger gas exchange amount.

In an embodiment, as shown in FIG. 1, the rotating portion is further provided with a rotating wheel 62. The reaction chamber body 10 is provided on the rotating wheel 62, and the rotating wheel 62 rotates with the rotation of the reaction chamber body 10. In this way, even if the reaction chamber body 10 has a very large weight, it can be well supported and rotated, and it is easier to realize large-scale in-vitro biological reaction production. As shown in FIG. 1, the rotating wheel 62 is provided on a frame 62a, and the reaction chamber body 10 is provided on the rotating wheel 62.

In an embodiment, the rotating portion includes a driving unit 63 that drives the reaction chamber body to rotate. As shown in FIG. 2, the driving unit 63 drives the reaction chamber body 10 to rotate through a rotating shaft.

In an embodiment, the reaction device 100 further includes a temperature regul ating device (not shown) configured to keep the in-vitro biosynthesis reaction under a predetermined temperature condition. For example, the reaction chamber body 10 can be kept warm, heated or cooled, etc. Preferably, the temperature regulating device is configured to adjust a temperature of the gas entering the reaction chamber body to maintain a predetermined temperature condition, for example, heat the gas entering the internal space

In this embodiment, an in-vitro biosynthesis method is also provided, which adopts the aforementioned reaction device 100 to accommodate a reaction liquid for synthesis to perform the in-vitro biosynthesis reaction. Specifically, the method is described in conjunction with the aforementioned reaction device 100.

In an embodiment, in the in-vitro biosynthesis method of this embodiment, a ratio of a volume of the reaction liquid for synthesis used for the in-vitro biosynthesis reaction to a volume of the reaction chamber body is less than or equal to 30%, which can ensure that the reaction liquid is sufficiently spread and ensure the reaction efficiency.

In an embodiment, in the in-vitro biosynthesis method of this embodiment, during the reaction of the in-vitro biosynthesis, an external gas, such as air, is introduced into the internal space, so as to facilitate the oxygen required for the reaction.

In an embodiment, in the in-vitro biosynthesis method of this embodiment, during the in-vitro biosynthesis reaction, the gas in the internal space is exhausted, so as to prevent waste gas generated by the reaction from affecting the synthesis.

In an embodiment, in the in-vitro biosynthesis method of this embodiment, during the in-vitro biosynthesis reaction, the reaction chamber body 10 of the reaction device is rotated, so that the reaction liquid is spread onto the inner wall and the partition member, and can be mixed. In addition, a rotation speed of the reaction chamber body 10 is less than or equal to 120 r/min, so that foam is not easy to generate, and the synthesis quality is ensured.

### Embodiment 2

In this embodiment, the same components as those in embodiment 1 are denoted by the same numbers, and the same descriptions and explanations are omitted.

FIG. 8 is an overall structural schematic view of a second structure according to the present disclosure.

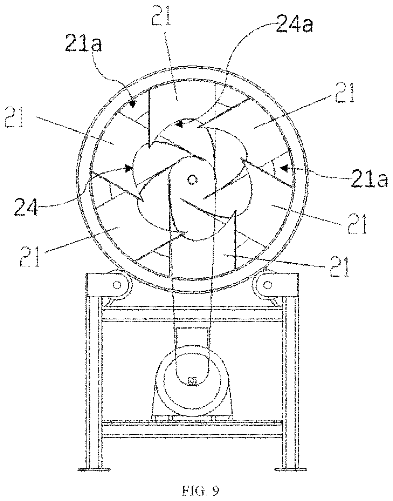

FIG. 9 is another schematic view of FIG. 8.

As shown in FIGS. 8 and 9, six partition members 21 are provided, which are evenly spaced apart, and a space area 21a is formed between every two adjacent partition members 21.

In an embodiment, as shown in FIG. 9, the other side edge 24 of the sheet-shaped partition member 21 away from the inner wall is provided with a recessed area 24a. In this way, when the partition member 21 is provided on the inner wall, which can facilitate the flow of the reaction liquid between the space areas, and improve the overall mixing efficiency.

In an embodiment, as shown in FIG. 8, an end of the gas intake duct 30a of the gas intake unit extends from the gas inlet into the internal space 11. In this case, preferably, at least one gas inlet duct extends in a horizontal direction X of the reaction chamber body, so that the incoming gas can reach as far as possible to the other end of the reaction chamber body 10 which is prone to lack of oxygen gas.

Further preferably, as shown in FIG. 8, a duct wall of a portion of the at least one gas intake duct 30a that enters the internal space 11 is provided with at least one gas outlet hole 31. That is, when a plurality of gas intake ducts 30a are provided, each gas intake duct partially enters the internal space. Among the gas intake ducts 30a, at least one gas outlet hole 31 is provided on the duct wall of the portion of the at least one gas intake duct 30a that enters the internal space 11. Preferably, the gas outlet holes 31 are evenly distributed.

In an embodiment, when the gas outlet hole is provided, an end of the gas intake duct 30a entering the internal space may be sealed.

In an embodiment, the exhaust unit includes a gas exhaust duct 41, and a portion of the gas intake duct 30a between the two ends of the gas intake duct is provided in the gas exhaust duct. That is, one end of the gas intake duct 30a extends out of one end of the gas exhaust duct 41 and extends into the internal space, and the other end of the gas intake duct 30a extends out of the other end of the gas exhaust duct 41 and is in communication with an air source. That is, a part of the gas inlet duct 30a is provided in the gas exhaust duct 41. In this way, the entering and exhausting of gas are separated, so that the gas intake duct and the gas exhaust duct can be provided at the same portion of the reaction chamber body. As shown in FIG. 8, the entering and discharging of gas are both provided at a left end.

In an embodiment, the reaction device 100 further includes a cover assembly having a cover body adapted to the feeding port to cover the feeding port.

Preferably, the cover assembly further includes a sealing ring, a tank cover latch, and a locking handle.

In an embodiment, the exhaust unit further includes a gas exhaust hole 42 provided on a cover body 51, and the gas exhaust duct 41 is in communication with the internal space through the gas exhaust hole 42.

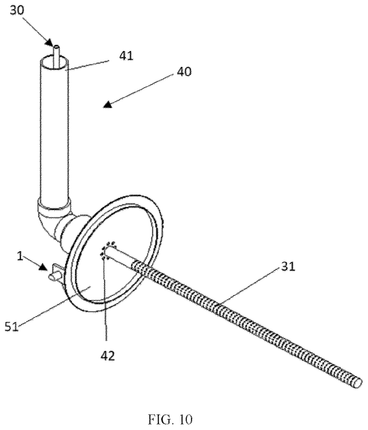

FIG. 10 is an assembly view of the gas intake unit, the gas exhaust unit, and a cover assembly according to the present disclosure.

In an embodiment, as shown in FIG. 10, the cover assembly 1, the gas intake unit 30, and the gas exhaust unit 40 have an integral structure. In this way, in use, one end of the gas intake duct 31 extends into the reaction body 10, and the other end of the gas intake duct 31 extends out of the gas exhaust duct 41 to be connected to the air source, and the cover body 51 provided with the gas exhaust hole 42 is adaptively mounted on the feeding port, i.e., the entire configuration is completed. The structure is ingenious, and the mounting is convenient.

### Embodiment 3

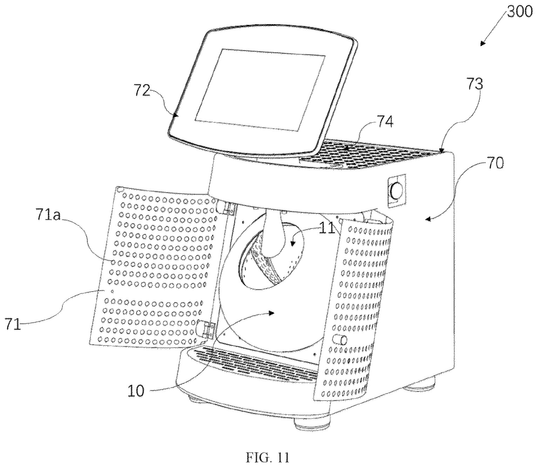

FIG. 11 is an overall structure schematic view of a reaction device of a third structure according to the present disclosure.

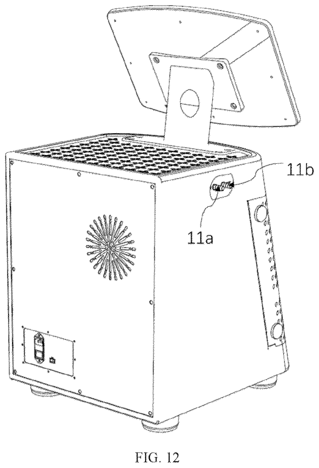

FIG. 12 is a side view of FIG. 11.

As shown in FIGS. 11 and 12, in this embodiment, the same components as those in embodiment 1 are denoted by the same numbers, and the same descriptions and explanations are omitted. The reaction device 300 of this embodiment further includes a housing 70 adapted to the reaction chamber body 10 and configured to accommodate the reaction chamber body 10.

Preferably, the housing 70 has any one or a combination of the following features:
(1) The housing 70 is provided with an opening and closing door 71. Preferably, the opening and closing door 71 is provided with a vent hole 71a, so that the gas in the internal space 11 of the reaction chamber body 10 can be exhausted or the external gas can enter the internal space 11.
(2) A display screen 72 is provided on an outer side of the housing 70, that is, not on an interior of the housing 70, but on the outer side, for example, on an external surface. Preferably, the display screen 72 is located on an outer surface of an upper top 73 of the housing 70. For another example, the display screen 72 is embedded in the outer surface. Through the display screen 72, for example, the display of the reaction process, the state, the condition, etc. can be achieved, and the reaction and the discharge of the reacted product can be completed by the touch operation.
(3) The outer side of the housing 70 is provided with an object placing platform configured to place objects. Preferably, an object placement surface of the object placing platform is provided with non-slip textures 74. Preferably, the outer surface of the upper top of the housing is the object placing platform.
(4) The housing is provided with any one or more of the following: a gas intake channel, a gas exhaust channel, a feeding channel, and a discharging channel. Specifically, the gas intake channel (not shown) is provided to allow the external air to enter the internal space 11 of the reaction chamber body 10, for example, the gas intake channel is in communication with the gas inlet through the aforementioned gas intake duct to allow the gas to enter into the reaction chamber body. The gas in the internal space of the reaction chamber body 10 is exhausted through the gas exhaust channel, for example, the gas can be exhausted by communicating the gas outlet with the gas exhaust channel through the aforementioned gas exhaust duct. The reaction liquid for synthesis is allowed to enter the internal space 11 of the reaction chamber body 10 for in-vitro biosynthesis reaction by providing a feeding channel 11a (as shown in FIG. 12). The reacted product is discharged by providing a discharging channel 11b (as shown in FIG. 12).

### Embodiment 4

FIG. 3 is a structural schematic view of the reaction chamber body according to the present disclosure.

As shown in FIG. 3, a bioreactor provided in this embodiment includes a reaction chamber body 10.

The reaction chamber body 10 is configured to accommodate reaction liquid for synthesis to perform an in-vitro biosynthesis reaction, and is provided with the opening portion 10a having the feeding port configured to allow the reaction liquid for synthesis to enter an internal space 11 of the reaction chamber body to perform a reaction and a discharging port configured to allow a reacted product to be discharged. The reaction liquid may be poured directly into the feeding port, or a reacted product may be poured out from the discharging port. The feeding and discharging can also be achieved through other methods, for example, a feeding duct may be used to transport the reaction liquid for synthesis from the feeding port to the internal space 11, or a discharging duct may be used to discharge the reacted product from the internal space 11.

In an embodiment, the opening portion 10a is further provided with a ventilation unit having the gas inlet and/or the gas outlet. The gas inlet is configured to allow external gas to enter the internal space of the reaction chamber body, so that oxygen-containing gas, such as air, can be introduced, thereby facilitating the biosynthesis reaction. The gas outlet is configured to allow the gas in the internal space to be exhausted, so that the gas generated during the reaction that is unfavorable to the reaction, such as ethanol, can be exhausted in time to avoid affecting the synthesis efficiency and quality.

In an embodiment, one or more of the feeding port, the discharging port, the intake port, and, the gas outlet are the same opening. In FIG. 1, the feeding port, the discharging port, the intake port, and the gas outlet are the same opening, that is, the opening portion 10a. In other embodiments, the feeding port, the discharging port, the intake port, and the gas outlet may be different openings, or some of them may be the same opening.

In an embodiment, the reaction chamber body 10 is the tank body as shown in FIG. 1. Since the tank body is spherical or ellipsoidal, compared with other shapes, a surface area of the inner wall is larger, the reaction liquid has more chances to be spread on the inner wall, and since there is no dead corner, it is not easy to generate foam during the reaction. Preferably, it is a horizontal tank body, that is, when the tank body is ellipsoidal, a long axis is in a horizontal direction, which avoids the deposition of the reaction liquid at the bottom and is more conducive to spreading than in the vertical direction.

The tank body may be made of stainless steel.

In an embodiment, during use, the reaction chamber body 10 is rotated to complete the reaction, so that the reaction liquid can be effectively spread on the inner wall to increase the efficiency of the in-vitro synthesis, and the degree to which the reaction liquid is spread can be adjusted by controlling a volume of the added reaction liquid. Preferably, a horizontal central axis of the reaction chamber body 10 is deviated from a horizontal state by a certain angle, that is, the reaction chamber body 10 is inclined, so that when the reaction chamber body 10 rotates, the reaction liquid can be more easily spread on more portions of the inner wall.

In an embodiment, the inner wall of the reaction chamber body 10 is provided with at least one first protrusion. Through the first protrusion, the surface area of the inner wall can be increased, thereby increasing a contact area between the inner wall and the reaction liquid, and increasing the degree to which the reaction liquid is spread.

In an embodiment, the bioreactor further includes a rotation mounting portion configured to be rotatably connected to a rotating portion that rotates the reaction chamber body. Preferably, the rotation mounting portion is located on the opposite side of the opening portion. After the rotation mounting portion is rotatably connected to the rotating portion, the opening portion is inclined upwards. In this way, the reaction liquid can be in better contact with and spread on the inner wall of the reaction chamber body 10 and the partition portion 20 during rotation, and the reaction liquid can not overflow even if the opening is large. In this way, when the opening is used as a feeding port or a discharging port, it is more convenient for feeding or discharging, and when the opening is used as a gas inlet or a gas outlet, it can ensure a larger gas exchange amount.

The bioreactor provided in this embodiment is simple in structure and easy to manufacture. The volume of the reaction chamber body is more than 10 L, even up to ten thousand liters, which is convenient for expansion. The in-vitro biosynthesis reaction can be completed through rotation, which is conducive to promotion and application.

### Embodiment 5

FIG. 5 is a schematic view for explaining that an internal space of the reaction chamber body according to the present disclosure is partitioned.

FIG. 6 is a structural schematic view of the reaction chamber body in which an internal space of the reaction device of the first structure according to the present disclosure is incompletely partitioned by the partition portion.

In FIG. 5, the part covered by oblique lines represents the partition.

The partition member 21 is configured to incompletely partition the internal space of the reaction chamber body 10 according to embodiment 4 (as shown in FIG. 6). (In FIG. 6, 10b refers to the first protrusion mentioned in embodiment 1.)

Explanation is made with reference to FIG. 5, in which a circle represents a section of the reaction chamber body, an interior of the circle represents an internal space, and an arc in a middle of the circle represents the partition. The "incompletely partition" is relative to "completely partition". The "completely partition" means that the entire internal space is partitioned into different uncommunicated spaces. That is, the partition spaces are completely partitioned from each other, for example, two spaces are formed by completely partitioning by a plate along a certain cross-section of the entire internal space. At this time, for example, on a left side of FIG. 5, a cross-section of the entire partition completely covers a cross-section of the internal space. The "incompletely partition" means that the internal space is incompletely partitioned. As shown on the right side of in FIG. 5, although left and right edges and a lower edge of the partition are respectively in contact with an inner side wall, an upper edge is not in contact with the inner side wall, such incomplete partition forms space areas that are in communication with each other. Specifically, as shown on the right side of FIG. 5, on the cross-section, the part covered by the partition is the space area, and the space areas are in communication with each other through a blank part thereof.

In this way, due to the incompletely partition, when the aforementioned reaction liquid is contained, the reaction liquid in a "flowing state" can flow from one space area to another space area, and the reaction liquid flowing through the partition member 21 will be blocked by the partition member 21 to increase the mixing intensity of the reaction liquid during the in-vitro synthesis reaction, thereby improving the reaction efficiency.

The "flowing state" herein means that the reaction liquid is not stationary, but at least partially moves relative to an inner wall and the reasons for this include, but are not limited to, the following:

First, it is caused only by the movement of the reaction chamber body 10, for example, only by the vibration, rotation, etc. of the reaction chamber body;

Second, it flows only by other external forces:
(a) For example, the reaction liquid is stirred by an external force, for example, the partition member 21 is rotated to stir the reaction liquid, so that the reaction liquid enters the flowing state;
(b) For another example, the reaction liquid may be subjected to fluctuations caused by the introduction of high-pressure airflow or ultrasonic vibration, so that the reaction liquid enters the flowing state.

Third, both the first and the second work, for example, when the reaction chamber body 10 rotates, the partition member 21 also rotates.

In addition, when the reaction liquid is in the flowing state, due to the blocking of the partition member 21, another effect is that the chance of the reaction liquid being contact with the inner wall of the reaction chamber body 10 is increased, the reaction liquid being contact with the inner wall has a chance of being spread on the inner wall under certain conditions, such as gravity or rotation, and the spread reaction liquid has a larger surface area and can be more fully contact with the air, thereby improving the in-vitro synthesis efficiency.

In an embodiment, as shown in FIG. 6, at least one partition member 21 is provided, so as to incompletely partition the internal space 11 to form a plurality of space areas, and the plurality of partition members 21 can increase the chance of the reaction liquid being mixed and spread on the inner wall.

FIG. 7 is a structural schematic view of the partition member of the present disclosure.

As shown in FIG. 7, in an embodiment, the partition member 21 is in a form of a sheet having two opposite sheet-shaped surfaces 22 and side edges 23 provided on both sides of the two opposite sheet-shaped surfaces. When the reaction liquid can be in contact with a sheet-shaped surface 22 of the partition member 21, the reaction liquid can also be spread onto the sheet-shaped surface 22. In this way, an area in which the reaction liquid of the same volume is spread is increased. Relatively speaking, the reaction liquid is also easier to be spread, and the contact area between the reaction liquid and the air is also increased, and the reaction efficiency is improved. The sheet-shaped surface 22 herein, as shown in FIG. 7, is relative to the side edge 23. In this case, the greater the number of partition members 21, the greater the area of the partition members 21 that can be in contact with the reaction liquid, and a spread area of the reaction liquid can be more increased, so that the contact area between the entire reaction liquid and the air is increased. Certainly, if the number of the partition members 21 is too large, a time for the spread reaction liquid to be contacted with the air each time may be too short, resulting in a longer reaction time, and if the number of the partition members 21 is too large, the overall weight is too large, which is not conducive to use, while the overall increase in the spreading is not too large, resulting in a waste of cost.

In an embodiment, the plurality of partition members 21 are evenly spaced apart, so that the internal space 11 can be incompletely partitioned into same space areas, and the spreading degree of the reaction liquid in each space area is equivalent. That is, the contact area of the reaction liquid is equivalent, the reaction efficiency is uniform, and the overall reaction effect is better.

In an embodiment, a length of the partition member 21 extends in a curved line or a folded line, which can increase the chance of contact between the reaction liquid and the partition member 21 in the same internal space, especially when the partition member is in a form of a sheet, the contact area can be further increased, thereby improving the synthesis efficiency, for example, improving the efficiency of the in-vitro protein synthesis reaction.

In an embodiment, as shown in FIG.6, an angle a is formed between a line 21A where a connecting line formed between two ends of the length of the partition member 21 is located and a central axis 11A of the internal space 11 of the reaction chamber body 10 in the same direction as the extending direction of the length.

Herein, "the central axis 11A of the internal space 11 of the reaction chamber body 10 in the same direction as the extending direction of the length" specifically means that when the length of the partition member 21 extends substantially in a length direction of the reaction chamber body 10, the central axis 11A is a central axis in the length direction of the reaction chamber body 10. When the length of the partition member 21 extends substantially in a width direction of the reaction chamber body 10, the central axis 11A is a central axis in the width direction of the reaction chamber body 10. The same direction herein means substantially the same, not absolutely the same.

The angle a herein means that the line 21A where the connecting line is located is not parallel to the central axis 11A, but is at an angle to the central axis 11A, that is, the line 21A is inclined to the central axis 11A. Preferably, the angle a is less than or equal to 60°, preferably less than or equal to 45°, and further preferably less than or equal to 20°. In this way, it is convenient for the reaction liquid in each space area formed by the separation to flow from one end to the other end of the reaction chamber body 10. In this way, when an end of the partition member 21 is in communication with the adjacent space area, it is convenient for the reaction liquid to flow from the end to the adjacent space area, thereby improving the mixing efficiency. The angle a should not be too large, that is, a connecting line 21a should not deviate too much from the axis, otherwise the reaction liquid will accumulate at one end of the reaction chamber body 10, which is not conducive to spreading.

In an embodiment, the partition portion 20 is provided on the inner wall of the reaction chamber body 10. In practice, the partition portion 20 may be rotatably fixed on the inner wall or non-rotatably fixed on the inner wall. When a plurality of partition members 21 are provided, the partition portions 20 may be all rotatably provided on the inner wall or partially rotatably provided on the inner wall. Preferably, when the partition member 21 is in a form of a sheet, one side edge 23 of the sheet-shaped partition member 21 faces the inner wall of the reaction chamber body 10, that is, the partition member 21 is rotatably or non-rotatably disposed on the inner wall by the rotatability or non-rotatability between the side edge 23 and the inner wall.

In an embodiment, when one side edge 23 of the sheet-shaped partition member 21 faces the inner wall of the reaction chamber body 10, an angle between a sheet-shaped surface of the sheet-shaped partition member and an opposite inner wall (referring to an inner wall where the side edge 23 is located) is not equal to 90°, which actually means that the sheet-shaped surface is not completely perpendicular to the inner wall, but deviates from a vertical plane by a certain angle b. In this way, compared with perpendicular, foam generated in the reaction can be reduced, the reaction effect can be improved, and the reaction liquid can be better spread.

In an embodiment, the length of the partition member 21 extends along the length direction of the reaction chamber body 10, and extends from one end to the other end in the length direction of the reaction chamber body, that is, the length of the partition member is substantially equivalent to the length of the reaction chamber body, which can ensure a larger contact area, thereby improving the synthesis efficiency.

FIG. 9 shows a side sectional view of a structure in which the reaction chamber body is incompletely partitioned by the partition member.

In an embodiment, as shown in FIG. 9, one side edge 24 of the sheet-shaped partition member 21 is provided with the recessed area 24a. In this way, when the partition member 21 is provided on the inner wall, the recessed area 24a is arranged away from the inner wall as shown in FIG. 9, which can facilitate the flow of the reaction liquid between the space areas, and improve the overall mixing efficiency.

In an embodiment, the partition member 21 is provided with at least one flow hole (not shown), so as to facilitate the reaction liquid to flow from one space area to another space area. Preferably, when a plurality of flow holes are provided, the flow holes on at least one partition member 21 are evenly arranged on the partition member 21, for example, three partition members 21 are provided, each partition member 21 is provided with a plurality of flow holes, and the flow holes on any one or more of the three partition members may be evenly distributed.

In an embodiment, as shown in FIGS. 2 and 4, at least one second protrusion 21b is provided on the outer surface of the partition member 21. Similarly, Similarly, the surface area of the partition member 21 can be increased by the second protrusion 21b, thereby increasing the contact area between the partition member 21 and the reaction liquid, and increasing the degree to which the reaction liquid is spread.

### Embodiment 6

In this embodiment, the same components as those in the foregoing embodiments are denoted by the same numbers, and the same descriptions and explanations are omitted.

FIG. 11 is an overall structure schematic view of a reaction device of the third structure according to the present disclosure.

FIG. 12 is a side view of FIG. 11.

As shown in the reaction device 300 in the figure, the housing 70 provided in this embodiment is adapted to the reaction chamber body 10 and is configured to accommodate the reaction chamber body 10.

Preferably, the housing 70 has any one or a combination of the following features:
(1) The housing 70 is provided with an opening and closing door 71. Preferably, the opening and closing door 71 is provided with a vent hole 71a, so that the gas in the internal space 11 of the reaction chamber body 10 can be exhausted or the external gas can enter the internal space 11.
(2) A display screen 72 is provided on an outer side of the housing 70, that is, not on an interior of the housing 70, but on the outer side, for example, on an external surface. Preferably, the display screen 72 is located on an outer surface of an upper top 73 of the housing 70. For another example, the display screen 72 is embedded in the outer surface. Through the display screen 72, for example, the display of the reaction process, the state, the condition, etc. can be achieved, and the reaction and the discharge of the reacted product can be completed by the touch operation.
(3) An outer side of the housing 70 is provided with an object placement platform configured to place an object. Preferably, an object placement surface of the object placement platform is provided with non-slip textures 74. Preferably, an outer surface of an upper top of the housing is the object placement platform.
(4) The housing is provided with any one or more of the following: a gas intake channel, a gas exhaust channel, a feeding channel, and a discharging channel. Specifically, the gas intake channel (not shown) is provided to allow the external gas to enter the internal space 11 of the reaction chamber body 10, for example, the gas intake channel is in communication with the gas inlet through the aforementioned gas intake duct to allow the gas to enter into the reaction chamber body. The gas in the internal space of the reaction chamber body 10 is exhausted through the gas exhaust channel, for example, the gas can be exhausted by communicating the gas outlet with the gas exhaust channel through the aforementioned gas exhaust duct. The reaction liquid for synthesis is allowed to enter the internal space 11 of the reaction chamber body 10 to perform the in-vitro biosynthesis reaction by providing a feeding channel 11a (as shown in FIG. 12). The reacted product is discharged by providing a discharging channel 11b (as shown in FIG. 12).

### Embodiment 7

FIG. 1 is an overall structural schematic view of a reaction device of a first structure according to the present disclosure;

FIG. 2 is another schematic view of FIG. 1.

As shown in FIG. 1 and FIG. 2, the reaction device 100 for in-vitro biosynthesis provided in this embodiment includes a bioreactor and the partition portion20. The bioreactor is the bioreactor of embodiment 4, and the partition portion 20 is provided with at least one partition member 21 in embodiment 5. The at least one partition member 21 performs the aforementioned incomplete partition of the internal space of the reaction chamber body 10 of the bioreactor.

In an embodiment, when the partition member 21 is provided on the inner wall, at least one discontinuity is provided between the partition member and the inner wall, that is, at least one gap is provided between the partition member 21 and the inner wall, which facilitates the reaction liquid to flow from one space area to another space area. Preferably, the discontinuities between the at least one partition member 21 and the inner wall are equally spaced apart. For example, three partition members 21 are provided on the inner wall, each partition member 21 has at least one discontinuity with the inner wall, and the discontinuities between at least one of the three partition members 21 and the inner wall are equally spaced apart.

In an embodiment, the reaction device 100 further includes a gas intake unit through which the aforementioned gas inlet allows the external gas to enter the internal space 11.

In an embodiment, the gas intake unit 30 includes at least one gas intake duct. As shown in FIG. 1, which shows one gas intake duct 30a, one end of which faces the gas inlet to deliver the external gas to the internal space, i.e., the gas intake duct has a distance from the internal space 11.

In an embodiment, the reaction device 100 further includes a gas exhaust unit through which the aforementioned gas outlet allows gas in the internal space 11 to be exhausted.

In an embodiment, the exhaust unit includes at least one gas exhaust duct that is in communication with the aforementioned gas outlet or extends into the internal space 11 to exhaust gas from the internal space 11.

In an embodiment, the reaction device 100 further includes a rotating portion configured to rotate the reaction body 10. Preferably, the rotating portion is rotatably connected to the reaction chamber body to rotate the reaction chamber body 10. Preferably, the rotating portion is rotatably connected to a rotation mounting portion of the reaction chamber body 10 located on an opposite side of the opening portion. After the rotating connection, an opening of the opening portion is inclined upward (as shown in FIG. 1). In this way, the reaction liquid can be in better contact with and spread on the inner wall of the reaction chamber body 10 and the partition portion 20 during rotation, and the reaction liquid can not overflow even if the opening is large. In this way, when the opening is used as a feeding port or a discharging port, it is more convenient for feeding or discharging, and when the opening is used as a gas inlet or a gas outlet, it can ensure a larger gas exchange amount.

In an embodiment, as shown in FIG. 1, the rotating portion is further provided with a rotating wheel 62. The reaction chamber body 10 is provided on the rotating wheel 62, and the rotating wheel 62 rotates along with a rotation of the reaction chamber body 10. In this way, even if the reaction chamber body 10 has a very large weight, it can be well supported and rotated, and it is easier to achieve large-scale in-vitro biological reaction production. As shown in FIG. 1, the rotating wheel 62 is provided on a frame 62a, and the reaction chamber body 10 is provided on the rotating wheel 62.

In an embodiment, the rotating portion includes a driving unit 63 that drives the reaction chamber body to rotate. As shown in FIG. 2, the driving unit 63 drives the reaction chamber body 10 to rotate through a rotating shaft.

In an embodiment, the reaction device 100 further includes a temperature regulating device (not shown) configured to maintain the in-vitro biosynthesis reaction under a predetermined temperature condition. For example, the reaction chamber body 10 can be kept warm, heated or cooled, etc. Preferably, the temperature regulating device is configured to regulate a temperature of the gas entering the reaction chamber body to maintain the predetermined temperature condition, for example, to heat the gas entering the internal space.

In an embodiment, the reaction device of this embodiment further includes a housing adapted to the reaction chamber body 10 and configured to accommodate the reaction chamber body. The housing is the housing of Embodiment 6.

In this embodiment, an in-vitro biosynthesis method is also provided, which adopts the aforementioned reaction device 100 to accommodate a reaction liquid for synthesis to perform the in-vitro biosynthesis reaction. Specifically, the method is described in conjunction with the aforementioned reaction device 100.

In an embodiment, in the in-vitro biosynthesis method of this embodiment, a ratio of a volume of the reaction liquid for synthesis configured to perform the in-vitro biosynthesis reaction to a volume of the reaction chamber body is less than or equal to 30%, which can ensure that the reaction liquid is sufficiently spread and ensure the reaction efficiency.

In an embodiment, in the in-vitro biosynthesis method of this embodiment, during the reaction of the in-vitro biosynthesis, an external gas, such as air, is introduced into the internal space, so as to facilitate the oxygen required for the reaction.

In an embodiment, in the in-vitro biosynthesis method of this embodiment, during the in-vitro biosynthesis reaction, the gas in the internal space is exhausted, so as to prevent waste gas generated by the reaction from affecting the synthesis.

In an embodiment, in the in-vitro biosynthesis method of this embodiment, during the in-vitro biosynthesis reaction, the reaction chamber body 10 of the reaction device is rotated, so that the reaction liquid is spread onto the inner wall and the partition member, and can be mixed. In addition, a rotation speed of the reaction chamber body 10 is less than or equal to 120 r/min, so that foam is not easy to generate, and the synthesis quality is ensured.

### Example 1

In this embodiment, the following are used to test an expression of a target protein EGFP using an in-vitro transcription and translation system:
(1) A first experimental device: the reaction device of the first structure is adopted, the inner wall of the reaction chamber body is provided with the first protrusion, and the outer surface of the partition is provided with the second protrusion.
(2) A second experimental device: the difference from the first experimental device lies in that the inner wall of the reaction chamber body and the outer surface of the partition member are smooth.
(3) A third experimental device: 24-well plate.

1 L of the same IVTT reaction liquid (in-vitro transcription and translation system, in-vitro transcription and translation coupled system) was added to the internal space of the reaction chamber body of each of the first experimental device and the second experimental device, respectively, and the biosynthesis reaction of in-vitro protein synthesis was performed at the same rotation speed and temperature.

300 uL of the same IVTT reaction liquid was added to the 24-well plate to perform the same biological reaction at the same temperature as a control.

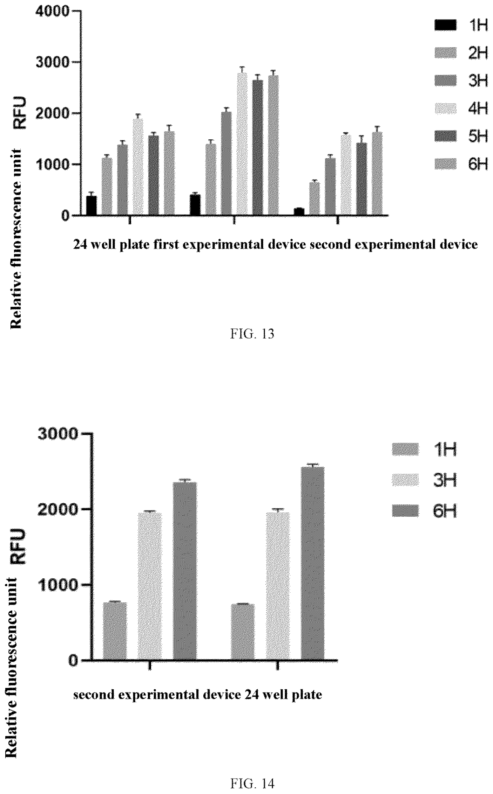

The above samples after reaction for 1h, 2h, 3h, 4h, 5h, and 6h respectively were taken to test the fluorescence value of EGFP. The results are shown in FIG. 13, which shows that the target protein expression activity of the reaction device provided by the present disclosure can be maintained compared with that of the 24-well plate, and the reaction device with the protrusion has a higher fluorescence value of the IVTT product, that is, the IVTT activity is better.

### Example 2

FIG. 14 shows activity test results of the target protein EGFP prepared by reacting for 1h, 3h, and 6h respectively by the in-vitro biosynthesis method of the present disclosure using the reaction device of the second structure. The volume of the reaction chamber body was 50 L, and the volume of the added IVTT reaction liquid was 10 L. The control group was the target protein prepared by using the 24-well plate in the laboratory, and 300 uL of the same IVTT reaction liquid was is added into the 24-well plate, and the two biological reactions of in-vitro protein synthesis were performed under the same rotational speed and temperature conditions.

As can be seen from FIG. 14, using the reaction device for in-vitro biosynthesis of the present disclosure, the activity of the target protein EGFP obtained at three reaction times is equivalent to that of the control group, and the activity is well maintained, which confirms that the reaction device for in-vitro biosynthesis according to the present disclosure can perform expanded production of in-vitro biosynthesis.

It should be noted that in the above embodiments, both gas intake and material feeding can be accomplished by pumping, and both gas exhaust and material discharging can be accomplished by suction.

## Claims

1. A reaction device for in-vitro biosynthesis, comprising:
a reaction chamber body configured to accommodate reaction liquid for synthesis to perform an in-vitro biosynthesis reaction, the reaction chamber body being provided with an opening portion having a feeding port configured to allow the reaction liquid for synthesis to enter the reaction chamber body and a discharging port configured to allow a reacted product to be discharged therefrom; and
a partition portion configured to incompletely partition an internal space of the reaction chamber body.

2. The reaction device according to claim 1, wherein the reaction chamber body is a tank body, preferably a horizontal tank body.

3. The reaction device according to claim 1 or 2, wherein the partition portion comprises at least one partition member,
preferably, the partition member is in a form of a sheet; and/or a plurality of the partition members are evenly spaced apart.

4. The reaction device according to any one of claims 1 to 3, wherein a length of the partition member extends in a form of a curved line or a folded line, and/or an angle *a* is formed between a connecting line formed between two ends of the length of the partition member and a central axis of an internal space of the reaction chamber body in the same direction as an extending direction of the length, preferably, preferably, the angle *a* is less than or equal to 60°, preferably, less than or equal to 45°, and further preferably, less than or equal to 20°.

5. The reaction device according to claims 1 to 4, wherein at least one partition member is rotatably and/or non-rotatably fixed on an inner wall of the reaction chamber body,
preferably, one side edge of the sheet-shaped partition member faces the inner wall of the reaction chamber body, and/or another side edge of the sheet-shaped partition member away from the inner wall is provided with a recessed area,
further preferably, when one side edge of the sheet-shaped partition member faces the inner wall of the reaction chamber body, an angle between a sheet-shaped surface of the partition member and the opposite inner wall is not equal to 90°.

6. The reaction device according to any one of claims 1 to 5, wherein in use, the reaction chamber body is rotated to perform the reaction,
preferably, a horizontal central axis of the reaction chamber body is deviated from a horizontal state.

7. The reaction device according to any one of claims 3 to 7, wherein the partition member is provided with at least one flow hole; and/or, when the partition member is provided on the inner wall, at least one discontinuity is provided between the partition member and the inner wall; preferably, the at least one flow hole on the partition member is evenly distributed, and/or the at least one discontinuity corresponding to the partition member is equally spaced apart.

8. The reaction device according to any one of claims 1 to 7, wherein the opening portion is further provided with a ventilation unit having a gas inlet and/or a gas outlet, the gas inlet is configured to allow an external gas to enter an internal space of the reaction chamber body, and the gas outlet is configured to allow the gas in the internal space to be exhausted;
preferably, one or more of the feeding port, the discharging port, the gas inlet, and the gas outlet are the same opening.

9. Thereaction device according to claim 8, further comprising a gas intake unit and/or a gas exhaust unit, wherein the gas inlet allows the external gas to enter the internal space through the gas intake unit, and the gas outlet allows gas in the internal space to be exhausted from the gas exhaust unit through the exhaust unit.

10. The reaction device according to claim 9, wherein the gas intake unit comprises at least one gas intake duct having an end facing the gas inlet to deliver the external air to the internal space,
or an end of the gas intake duct extends into the internal space from the gas inlet, at this time, preferably, at least one gas intake duct extends along a horizontal direction of the reaction chamber body; more preferably, a duct wall of a portion of the at least one gas intake duct that enters the internal space is provided with at least one gas exhaust hole, preferably, the at least one gas exhaust hole is evenly distributed, and/or one end of the gas intake duct entering the internal space is sealed.

11. The reaction device according to claim 10, wherein the gas exhaust unit comprises a gas exhaust duct, preferably, a portion of the gas intake duct between two ends of the gas intake duct is provided in the gas exhaust duct, one end of the gas intake duct extends out of one end of the gas exhaust duct and extends into the internal space, and another end of the gas intake duct extends out of another end of the gas exhaust duct and is in communication with an air source.

12. The reaction device according to any one of claims 1 to 11, further comprising:
a cover assembly having a cover body adapted to the feeding port and configured to cover the feeding port, wherein, preferably, the cover assembly further comprises a sealing ring, a tank cover latch, and a locking handle.

13. The reaction device according to claim 12, wherein when claim 12 depends on claim 11, the gas exhaust unit further comprises a gas exhaust hole provided on the cover body, and the gas exhaust duct is in communication with the internal space through the gas exhaust hole.

14. The reaction device according to any one of claims 1 to 13, further comprising:
a rotating portion configured to rotate the reaction chamber body,
wherein, preferably, the rotating portion is rotatably connected to the reaction chamber body to rotate the reaction chamber body;
and/or the rotating portion is further provided with a rotating wheel, the reaction chamber body is provided on the rotating wheel, and the rotating wheel rotates along with a rotation of the reaction chamber body.

15. The reaction device according to claim 14, wherein the rotating portion comprises a driving unit that drives the reaction chamber body to rotate.

16. The reaction device according to any one of claims 1 to 15, further comprising:
a temperature regulating device configured to maintain the reaction under a predetermined temperature condition;
preferably, the temperature regulating device is configured to regulate a temperature of gas entering the reaction chamber body to maintain the predetermined temperature condition.

17. The reaction device according to any one of claims 1 to 16, wherein an inner wall of the reaction chamber body is provided with at least one first protrusion, and/or an outer surface of the partition member is provided with at least one second protrusion.

18. The reaction device according to any one of claims 1 to 17, further comprising:
a housing adapted to the reaction chamber body and configured to accommodate the reaction chamber body, wherein, preferably, the housing has any one or a combination of the following features:
the housing is provided with an opening and closing door, and preferably, the opening and closing door is provided with a vent hole;
a display screen is provided on an outer side of the housing, and preferably, the display screen is located on an outer surface of an upper top of the housing;
an outer side of the housing is provided with an object placing platform configured to place an object, preferably, an object placing surface of the object placing platform is provided with non-slip textures, and more preferably, an outer surface of an upper top of the housing is the object placing platform;
the housing is provided with any one or more of: a gas intake channel configured to allow external gas to enter the internal space of the reaction chamber body, a gas exhaust channel configured to allow gas in the internal space of the reaction chamber body to be exhausted, a feeding channel configured to allow the reaction liquid for synthesis to enter the internal space of the reaction chamber body to perform the in-vitro biosynthesis reaction, and a discharging channel through which the reacted product is discharged.

19. An in-vitro biosynthesis method, comprising:
using the reaction device according to any one of claims 1 to 18 to accommodate the reaction liquid for synthesis to perform the in-vitro biosynthesis reaction.

20. The in-vitro biosynthesis method according to claim 19, wherein a ratio of a volume of the reaction liquid for synthesis configured to perform the reaction to a volume of the reaction chamber body is less than or equal to 30%.

21. The in-vitro biosynthesis method according to claim 19 or 20, wherein during the reaction, an external gas is introduced into the internal space, and/or gas in the internal space is exhausted

22. That in-vitro biosynthesis method according to any one of claims 19 to 21, wherein during the reaction, the reaction chamber body of the reaction device is rotated, and preferably, a horizontal central axis of the reaction chamber body is deviated from a horizontal state;
more preferably, a speed of rotation is less than or equal to 120 r/min.

23. A bioreactor for an in-vitro biosynthesis reaction, comprising:
a reaction chamber body configured to accommodate reaction liquid for synthesis to perform an in-vitro protein synthesis reaction, wherein the reaction chamber body is provided with an opening portion having a feeding port, a discharging port, and a ventilation unit, the feeding port is configured to allow the reaction liquid for synthesis to enter the reaction chamber body, the discharging port is configured to allow a reacted product to be discharged from the reaction chamber body, the ventilation unit is provided with a gas inlet and/or a gas outlet, the gas inlet is configured to allow external gas to enter an internal space of the reaction chamber body, and the gas outlet is configured to allow gas in the internal space to be exhausted.

24. The bioreactor according to claim 23, wherein an inner wall of the reaction chamber body is provided with at least one first protrusion.

25. The bioreactor according to claim 23 or 24, wherein one or more of the feeding port, the discharging port, the gas inlet, and the gas outlet are the same opening.

26. The bioreactor of claim 25, further comprising:
a rotation mounting portion configured to be rotatably connected to a rotating portion that rotates the reaction chamber body, preferably, the rotation mounting portion is located on an opposite side of the opening portion,and after the rotation mounting portion is rotatably connected to the rotating portion, the opening portion is inclined upwards.

27. The bioreactor according to any one of claims 23 to 25, wherein the reaction chamber body is a tank body.

28. A partition member for a biological reaction, configured to incompletely partition an internal space of a reaction chamber body of the bioreactor according to any one of claims 23 to 27.

29. The partition member according to claim 28, wherein a length of the partition member extends in a curved line or a folded line.

30. The partition member according to claim 28 or 29, wherein the partition member is in a form of a sheet having two opposite sheet-shaped surfaces and side edges provided on both sides of the two opposite sheet-shaped surfaces.

31. The partition member according to claim 30, wherein one of the side edges of the partition member is provided with a recessed area.

32. The partition member according to any one of claims 28 to 31, wherein the partition member is provided with at least one flow hole.

33. The partition member according to any one of claims 28 to 32, wherein an outer surface of the partition member is provided with at least one second protrusion.

34. A housing adapted to the reaction chamber body of the bioreactor according to any one of claims 23 to 27 and configured to accommodate the reaction chamber body.

35. The housing according to claim 34 having any one or a combination of the following features:
the housing is provided with an opening and closing door, and preferably, the opening and closing door is provided with a vent hole;
a display screen is provided on an outer side of the housing, and preferably, the display screen is located on an outer surface of an upper top of the housing;
an outer side of the housing is provided with an object placing platform configured to place an object, preferably, an object placing surface of the object placing platform is provided with non-slip textures, and more preferably, an outer surface of an upper top of the housing is the object placing platform;
the housing is provided with any one or more of: a gas intake channel configured to allow external gas to enter the internal space of the reaction chamber body, a gas exhaust channel configured to allow gas in the internal space of the reaction chamber body to be exhausted, a feeding channel configured to allow the reaction liquid for synthesis to enter the internal space of the reaction chamber body to perform the in-vitro biosynthesis reaction, and a discharging channel through which a reacted product is discharged.

36. A reaction device, comprising the bioreactor according to any one of claims 23 to 27 and at least one partition member according to any one of claims 28 to 33, wherein the at least one partition member incompletely partitions an internal space of the reaction chamber body of the bioreactor, and the bioreactor is integrally formed with the partition member.

37. The reaction device according to claim 36, further comprising the housing according to claim 34 or 35.
